Europäisches Patentamt

European Patent Office

Office européen des brevets

(19)

(11) Numéro de publication : **0 078 212**
**B1**

(12)

# FASCICULE DE BREVET EUROPÉEN

(45) Date de publication du fascicule du brevet :
28.08.85

(21) Numéro de dépôt : 82401946.7

(22) Date de dépôt : 22.10.82

(51) Int. Cl.⁴ : **G 01 N 1/10, G 21 F 7/06**

(54) **Banc de prélèvement d'échantillons liquides.**

(30) Priorité : 26.10.81 FR 8120039

(43) Date de publication de la demande :
04.05.83 Bulletin 83/18

(45) Mention de la délivrance du brevet :
28.08.85 Bulletin 85/35

(84) Etats contractants désignés :
BE DE GB IT SE

(56) Documents cités :
DE-A- 2 642 065
FR-A- 1 401 298
FR-A- 1 401 405
FR-A- 1 437 674
FR-A- 1 559 181
FR-A- 2 058 751
US-A- 4 120 662

(73) Titulaire : COMMISSARIAT A L'ENERGIE ATOMIQUE
Etablissement de Caractère Scientifique Technique
et Industriel
31/33, rue de la Fédération
F-75015 Paris (FR)
BE DE GB IT
SOCIETE GENERALE POUR LES TECHNIQUES NOU-
VELLES S.G.N. Société anonyme dite:
1, rue des Hérons Montigny-le-Bretonneux
F-78184 Saint-Quentin en Yvelines Cedex (FR)
SE

(72) Inventeur : Conche, François
15, rue du Château
F-78130 Les Mureaux (FR)
Inventeur : Naujalis, Pierre
16, Avenue Pierre Curie
F-94310 Orly (FR)

(74) Mandataire : Mongrédien, André et al
c/o BREVATOME 25, rue de Ponthieu
F-75008 Paris (FR)

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un banc de prélèvement d'échantillons liquides et notamment d'échantillons liquides radioactifs.

Le prélèvement à distance des échantillons de solutions actives dans les unités chimiques de traitement des combustibles irradiés est obtenu au moyen de dispositifs appelés « bancs de prise d'échantillons » ou « bancs de prélèvement ».

Les réservoirs parfois encore dénommés « capacités », contenant les liquides radioactifs à échantillonner sont en principe situés sous une dalle de protection biologique, dont la partie supérieure sert de plan de travail, ces réservoirs étant situés à des distances pouvant atteindre plusieurs dizaines de mètres. De ces réservoirs, le liquide à échantillonner est remonté au niveau du banc de prise d'échantillons et circule de préférence en continu. L'obturateur en matière souple et élastique d'un récipient dénommé « cruchon », préalablement mis sous vide, est piqué à l'extrémité d'une aiguille reliée à la veine de liquide à échantillonner. Après remplissage, le cruchon est transféré du banc de prise d'échantillons vers un laboratoire d'analyses.

De tels bancs sont décrits dans les brevets français FR-A-1.401.298, 1.401.405 et dans le certificat d'addition FR-A-2.058.751 au brevet français FR-A-1.401.298. Ces bancs connus comportent essentiellement, à l'intérieur d'une boîte à gants, une série d'arrivées de liquides au moyen d'embouts d'aiguilles creuses reliées à des réservoirs contenant des liquides radioactifs à doser, des moyens d'introduction des cruchons dans la boîte à gants et d'expulsion de ces cruchons de la boîte à gants, des moyens de manipulation permettant notamment de déplacer les cruchons à l'intérieur de la boîte à gants. Les moyens de manipulation sont fixés sur une porte-outil et sont généralement de trois types : un outil de débouchage des embouts d'arrivée d'aiguilles, un outil de prise de cruchons proprement dit, et un outil de démontage et remontage des aiguilles.

Dans ces bancs, les extrémités desdits embouts sont situées suivant au moins un arc de circonférence. Au-dessus de ces embouts et à l'intérieur de la boîte à gants, est disposé un support rotatif autour d'un premier axe. Sur ce support peut tourner un porte-outil autour d'un deuxième axe différent du premier, ce porte-outil pouvant en outre se déplacer le long de ce deuxième axe. Sur ce porte-outil sont placés, dans des positions excentrées parallèles au deuxième axe et à égale distance de cet axe, les outils à utiliser pour la prise d'échantillons.

Dans le certificat d'addition FR-A-2.058.751, on décrit plusieurs perfectionnements, dont l'un a pour objet un embout à aiguille courte de changement aisé.

Ces bancs d'échantillons fonctionnent correctement, mais ils présentent un certain nombre d'inconvénients. Tout d'abord, les bancs du type ci-dessus sont disposés à l'intérieur d'une boîte à gants, elle-même située au-dessus de la dalle de béton de protection biologique. Pour la protection contre les particules alpha, l'ensemble des panneaux de la boîte à gants et des outils est monté avec des joints d'étanchéité. En outre, pour se protéger des rayons gamma, il est nécessaire que la partie mécanique supérieure des outils soit constituée de pièces massives correspondant à une protection de 7 cm au moins de plomb. Par ailleurs, le pourtour du banc est entouré de plaques de plomb et sur la face, côté travail, on prévoit un hublot et des pinces d'intervention dans la boîte à gants. Dans ce cas, la boîte à gants et sa protection biologique forment un ensemble encombrant et très pesant.

Dans le certificat d'addition FR-A-2.058.751, on propose d'extraire automatiquement le cruchon rempli, et de le transporter hors du banc cuve au moyen d'un système de transfert pneumatique. Pour cela, on décrit un dispositif mécanique de conditionnement et d'évacuation du cruchon permettant automatiquement de confiner le cruchon dans un curseur et d'évacuer pneumatiquement l'ensemble cruchon-curseur. Ces dispositifs mécaniques sont assez complexes, et il est souhaitable d'en limiter le nombre en vue d'augmenter la fiabilité de l'ensemble de l'appareil.

D'autre part, dans les bancs ci-dessus les embouts ne sont pas disposés verticalement mais suivant une certaine inclinaison. Les outils présentent également une certaine inclinaison par rapport à la verticale et opèrent suivant un axe oblique. Cette disposition rendue nécessaire par la conception même des bancs à plusieurs embouts décrits dans le brevet ci-dessus nécessite un positionnement précis et une construction mécanique de précision dont la fiabilité peut être insuffisante. Enfin, lorsque le cruchon, après remplissage, est retiré de l'embout de l'aiguille, il subsiste une goutte sur la surface extérieure de l'obturateur souple et élastique percé par l'aiguille et cette goutte peut être une source dangereuse de contamination.

La présente invention se rapporte à un banc de prélèvement qui élimine ces inconvénients grâce à la suppression de la boîte à gants et dans lequel le nombre de dispositifs mécaniques est limité.

Le banc de prélèvement d'échantillons liquides, selon l'invention, est défini et caractérisé par la revendication 1 de la présente demande.

Selon un mode de réalisation préféré de l'invention, la dalle comprend un bouchon tournant situé au-dessus de la cuve précitée, les embouts d'aiguilles étant disposés suivant un arc de cercle ou plusieurs arcs de cercle concentriques tels que l'axe de rotation du bouchon passe par les centres de ces cercles. Dans ce cas, le ou les dispositifs mécaniques pour l'introduction et la manipulation des cruchons traversent le bouchon tournant et sont munis d'au moins un porte-outil situé à une distance de l'axe de rotation du bouchon égale au rayon des cercles définis ci-dessus. Pour plus de commodité, et afin de permettre leur transport par un système pneumatique, les cruchons sont disposés à l'intérieur de récipients appelés « curseurs » et ce sont les ensembles cruchon-curseur qui se déplacent à l'intérieur du banc.

Selon un autre aspect du banc de prélèvement objet de l'invention, chaque dispositif mécanique de manipulation des cruchons comprend au moins une vis hélicoïdale disposée verticalement, libre en rotation et immobilisée en translation, permettant de déplacer un ensemble cruchon-curseur le long d'un puits de descente, un outil de préhension, et un barillet permettant de déplacer l'ensemble cruchon-curseur entre le puits de descente et l'outil de préhension.

D'autres particularités et avantages de l'invention ressortiront de la description qui va suivre, donnée à titre d'exemple purement illustratif et nullement limitatif, en référence aux dessins annexés, dans lesquels :

la figure 1 est une vue schématique en coupe verticale d'un banc de prélèvement conforme à l'invention,

la figure 2 est une vue de dessus d'un dispositif mécanique d'introduction et de manipulation des cruchons,

la figure 3 est une vue agrandie en coupe verticale suivant la ligne III-III de la figure 2,

la figure 4 est une vue schématique en coupe de la partie supérieure d'un dispositif mécanique de manipulation des cruchons montrant l'introduction d'un ensemble cruchon-curseur dans ce dispositif,

la figure 5 est une vue schématique en coupe montrant un tel ensemble dans la partie inférieure du dispositif mécanique,

la figure 6 est une vue schématique montrant un cruchon disposé à l'intérieur d'un curseur, et

la figure 7 est une vue schématique en coupe montrant un mode de réalisation particulier d'un bouchon élastique pour un cruchon de prélèvement.

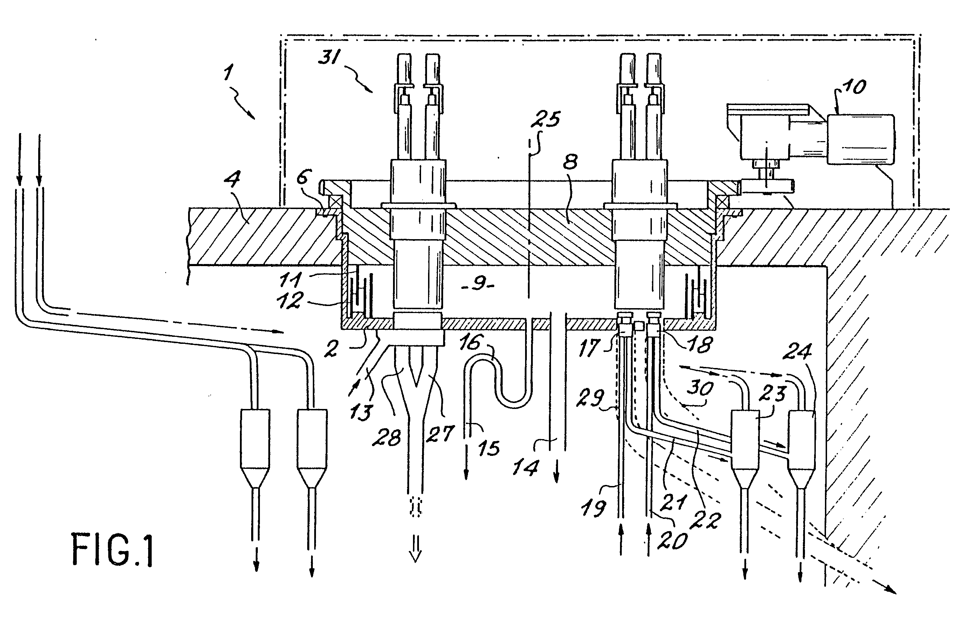

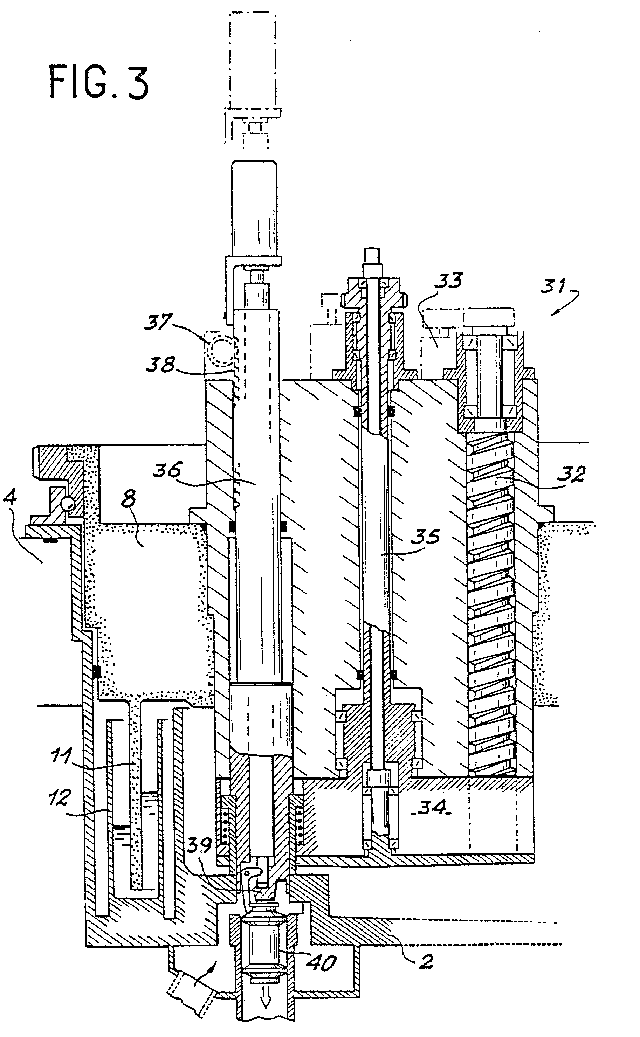

Sur la figure 1, on voit que le banc de prélèvement selon l'invention, portant la référence générale 1, comprend tout d'abord une cuve 2 fixée sous une dalle de protection 4 et solidaire de celle-ci : la cuve 2 est donc en zone active. Elle est soudée sur une couronne circulaire 6 prenant appui sur la partie supérieure de la dalle 4. Au-dessus de la cuve 2 est disposé un bouchon tournant 8 qui peut être mis en mouvement grâce à un moteur électrique 10. On délimite ainsi un espace 9 entre le bouchon 8 et la cuve 2. A la partie inférieure du bouchon 8 est fixé un manchon circulaire 11 qui plonge dans un récipient annulaire 12 lui-même solidaire de la cuve 2 et situé à la périphérie de celle-ci. Ce dispositif apparaît mieux sur la vue agrandie de la figure 3. On voit que le récipient 12 est rempli d'un liquide dans lequel plonge le manchon 11, ce qui constitue une garde hydraulique et assure l'étanchéité. Comme l'intérieur de la cuve 2 est mis en dépression, le niveau du liquide dans le récipient 12 est plus élevé du côté intérieur du manchon 11 que du côté extérieur. Le fond de la cuve 2 est en communication avec une tuyauterie 15 de vidange d'effluents munie d'un siphon 16 ou reliée à une tuyauterie plongeant dans une autre cuve. Sur le fond de la cuve 2 on voit deux embouts d'aiguille creuse, 17 et 18, reliés par des canalisations 19 et 20, respectivement, à un réservoir ou capacité de stockage de produits radioactifs (non représenté). Les embouts d'aiguille 17 et 18 sont reliés par deux canalisations de sortie 21 et 22 aux séparateurs 23 et 24 de deux dispositifs de mise en circulation de liquide par air-lift.

Les embouts d'aiguille peuvent être placés sur des supports munis chacun d'une conduite d'arrivée et d'une conduite de sortie du liquide à échantillonner séparées par une capacité intermédiaire (ou récipient intermédiaire) afin d'assurer une bonne homogénéisation du liquide. Avantageusement, on peut prévoir une conduite supplémentaire, ou canalisation de vidange, fortement inclinée et reliant la partie inférieure de la capacité intermédiaire à la conduite d'arrivée, permettant ainsi une vidange efficace du dispositif. On peut encore prévoir un manchon cylindrique ouvert à son extrémité inférieure et prolongeant l'embout d'aiguille à l'intérieur de la capacité intermédiaire : ce manchon joue un rôle de tranquilliseur afin de protéger l'aiguille contre les turbulences pouvant se produire au sein du liquide.

Les embouts 17 et 18 sont situés sur des arcs de cercle par le centre desquels passe l'axe de rotation 25 du bouchon tournant 8. Le fond de la cuve 2 comporte encore deux orifices de départ des ensembles cruchon-curseur par les canalisations 27 et 28 vers l'unité d'analyse. Dans une variante, les ensembles cruchon-curseur sont renvoyés vers l'unité d'analyse par les dispositifs d'introduction. Dans le cas particulier décrit ici, le dispositif comporte un certain nombre d'embouts tels que 17 répartis sur un premier arc de cercle et un certain nombre d'embouts tels que 18 répartis sur un deuxième arc de cercle concentrique au premier. Les canalisations 27 et 28 débouchent dans le fond de la cuve en des points situés sur les cercles définis par les embouts d'aiguille, à savoir le cercle défini par les embouts tels que 17 pour la canalisation 27 et le cercle défini par les embouts tels que 18 pour la canalisation 28. Le fond de la cuve 2 comporte également deux orifices d'évacuation des déchets, reliés à deux canalisations 29 et 30 représentées en traits interrompus sur la figure et situés sur les deux cercles précédemment définis. On voit encore deux dispositifs mécaniques d'introduction et de manipulation des cruchons tels que 32 disposés verticalement à travers le bouchon 8 et aboutissant à l'intérieur de la cuve 2.

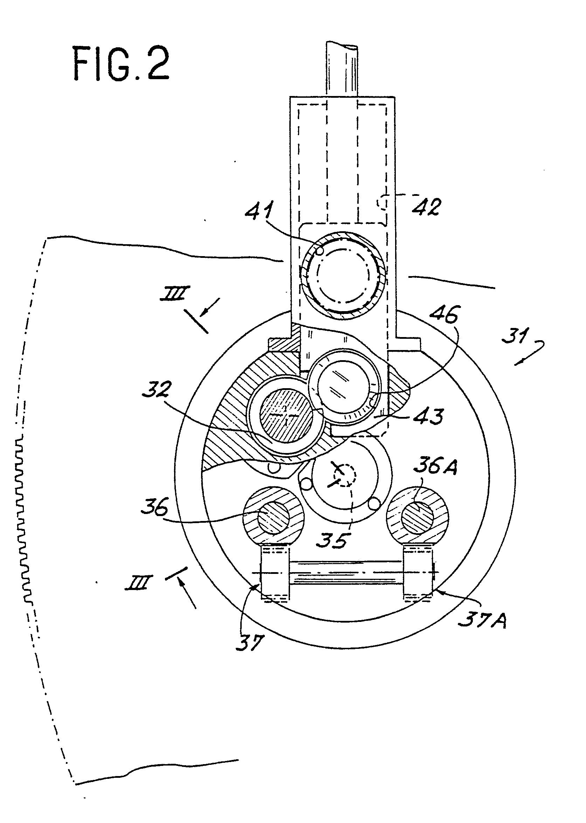

La partie supérieure d'un tel dispositif apparaît sur la vue de dessus de la figure 2. Un tube 41 permet à un ensemble cruchon-curseur d'arriver dans une chambre 42 où un piston 43 le pousse jusqu'au contact d'une vis hélicoïdale 32 dont le mouvement lui permet de descendre jusqu'au fond d'un puits de descente 46. Un plateau solidaire d'un barillet mobile autour de l'axe 35 permet d'amener l'ensemble cruchon-curseur sous l'un ou l'autre des porte-outils 36, 36a, mobiles verticalement grâce à des crémaillères coopérant avec des pignons 37, 37a.

Ces dispositifs apparaissent sur la vue en coupe de la figure 3. On voit la vis 32, immobilisée en translation, mais entraînée en rotation par un moteur 33.

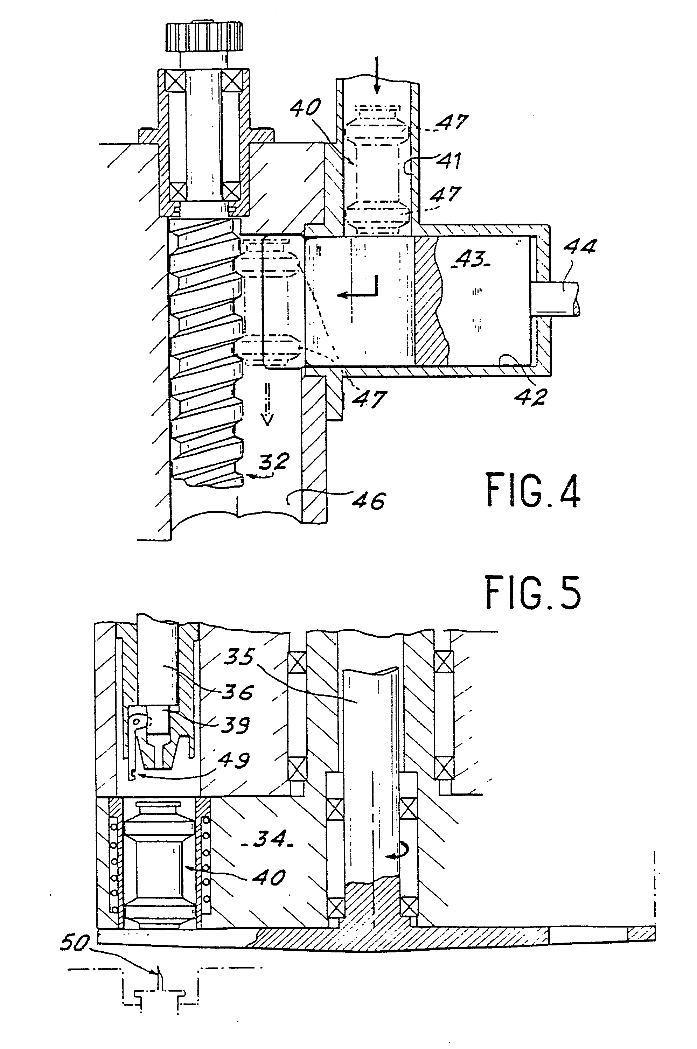

Le mécanisme d'introduction des cruchons dans le puits de descente sera décrit ultérieurement en référence à la figure 4. A la partie inférieure du dispositif se trouve un barillet 34 mobile autour d'un axe 35 et permettant d'amener l'ensemble cruchon-curseur de la partie inférieure du puits de descente à une position située sous le porte-outil 36 (figure 3). Ce dernier est mobile verticalement grâce à une crémaillère 38 coopérant avec le pignon 37. La partie inférieure du porte-outil 36 est munie d'un outil de préhension 39 permettant d'agripper l'ensemble cruchon-curseur et de l'amener au droit d'un embout d'aiguille préalablement sélectionné. La position des porte-outils à l'intérieur des dispositifs tels que 31 et la position de ces derniers dans le bouchon 8 sont déterminées de sorte que, par rotation du bouchon, on puisse amener un porte-outil au-dessus de n'importe quel embout d'aiguille. Dans le cas où ces derniers sont disposés suivant deux arcs de cercle concentriques, comme c'est le cas dans l'exemple décrit ici, les deux porte-outils 36, 36A sont identiques et situés à des distances de l'axe de rotation du bouchon égales au rayon des arcs de cercle définis par les embouts d'aiguilles 18 et 17 respectivement.

L'introduction d'un cruchon dans le dispositif 31 va être maintenant décrite en détail en référence à la figure 4. L'ensemble cruchon-curseur 40 est amené par transfert pneumatique dans un tube 41 débouchant dans une chambre 42. Un piston 43 commandé par une tige 44 peut se déplacer à l'intérieur de la chambre 42. Lorsqu'il est en position rétractée, l'ensemble 40 tombe dans la chambre 42 puis le piston 43 est poussé en avant afin d'amener l'ensemble cruchon-curseur 40 au sommet du puits de descente 46. Cependant, l'ensemble ne tombe pas dans le puits, car le curseur de l'ensemble cruchon-curseur (40) est muni à ses deux extrémités de renflements 47 coopérant avec les parties en creux du filetage de la vis 32. Cette dernière étant immobilisée en translation, sa mise en rotation assure la descente progressive de l'ensemble 40 jusqu'au barillet 34 qui se trouve à la partie inférieure du dispositif 31 (figure 5). Par rotation de celui-ci autour de l'axe 35, l'ensemble cruchon-curseur 40 est amené au-dessous du porte-outil 36. Ce dernier est alors descendu jusqu'à ce que la pince 49 de l'outil 39 puisse agripper l'ensemble cruchon-curseur 40 et piquer celui-ci sur l'aiguille 50. Une fois le prélèvement effectué, le porte-outil 36 remonte l'ensemble cruchon-curseur et, par un mouvement approprié du bouchon tournant, le porte-outil 36 est amené au droit d'un point de départ pneumatique des ensembles cruchon-curseur, par exemple au-dessus de la canalisation 28 (figure 1).

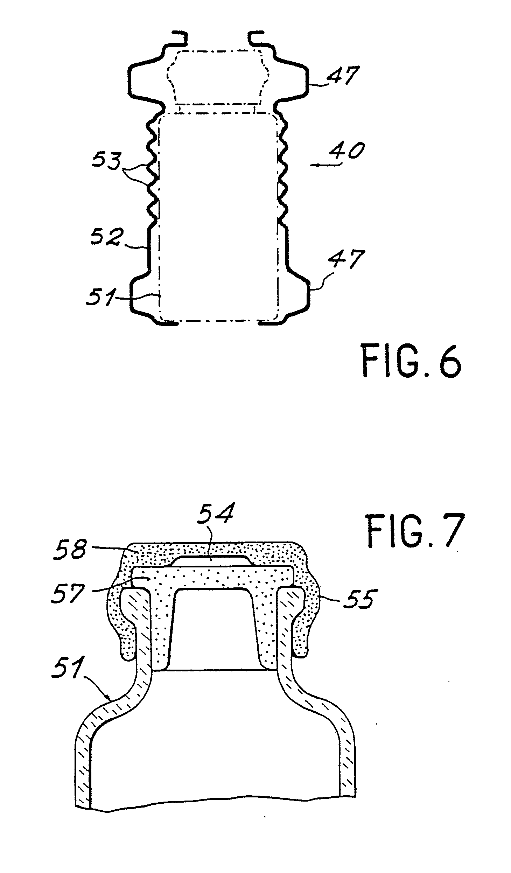

La figure 6 représente un ensemble cruchon-curseur utilisé dans le banc de prélèvement selon l'invention. Cet ensemble 40 comporte un cruchon 51 en polyéthylène logé en force dans un manchon cylindrique 52 ou curseur ouvert à ses deux extrémités et comportant sur sa paroi latérale un certain nombre de bossages circulaires 53, ce qui permet d'emmancher en force le cruchon 51 dans le curseur 52. De plus, celui-ci comporte à ses deux extrémités des renflements 47 coopérant avec le filetage de la vis hélicoïdale 32, ce qui permet la descente de l'ensemble dans le puits 46.

La figure 7 illustre un mode particulier de réalisation du bouchon élastique 55 du cruchon 51. Après le prélèvement, lorsqu'on retire le bouchon de l'aiguille, il reste généralement une goutte à l'extérieur du bouchon, et celle-ci peut être une source dangereuse de contamination. Afin d'éviter un tel risque, le bouchon 55 du cruchon 51 comporte un alvéole 54 entièrement fermé, dans lequel la goutte est retenue lorsque le cruchon est retiré de l'aiguille. Le bouchon 55 peut par exemple être réalisé en deux parties 57 et 58 collées ensemble, et dont seule la partie 58 comporte une cavité 54.

Le banc de prélèvement selon l'invention présente de nombreux avantages dont le principal est la simplicité puisque, du fait que les embouts d'aiguille sont situés dans la cuve 2 placée sous la dalle de protection, il n'y a plus besoin de boîte à gants. D'autre part, les dispositifs mécaniques de manipulation des cruchons ont leurs organes de commande situés à l'extérieur de la cuve 2, ce qui facilite les contrôles de positionnement. Ils sont également plus simples que dans les bancs de prélèvement de l'art antérieur, puisque le même outil peut servir aussi bien à la manipulation des cruchons qu'à l'enlèvement des bouchons protecteurs des embouts d'aiguille et au remplacement de ceux-ci.

## Revendications

1. Banc de prélèvement d'échantillons liquides à l'aide d'un récipient ou cruchon fermé (51) par un bouchon élastique (55) que l'on pique sur l'une de plusieurs aiguilles creuses munies d'embouts (17, 18) et reliées à un réservoir contenant le liquide à échantillonner, ce réservoir étant situé sous une dalle de protection biologique (4), caractérisé en ce qu'il comprend une cuve fermée (2) située sous la dalle de protection (4), solidaire de celle-ci et dans laquelle se trouvent les embouts (17, 18) desdites aiguilles creuses, et au moins un dispositif mécanique (31) pour la manipulation et l'introduction dudit cruchon (51) dans ladite cuve fermée (2), au moins une partie inférieure dudit dispositif mécanique (31) traversant la dalle (4) et aboutissant dans la cuve fermée (2) au niveau des embouts d'aiguilles (17, 18).

2. Banc de prélèvement selon la revendication 1, caractérisé en ce que les embouts d'aiguilles (17, 18) sont disposés suivant un arc de cercle ou plusieurs arcs de cercle concentriques.

3. Banc de prélèvement selon la revendication 2, caractérisé en ce que la dalle (4) comporte un bouchon tournant (8) dont l'axe de rotation (25) passe par le centre du ou des arcs de cercles définis par les embouts d'aiguilles.

4. Banc de prélèvement selon la revendication 3, caractérisé en ce qu'il comprend au moins un dispositif mécanique (31) d'introduction et de manipulation des cruchons (51, 52) dont la partie inférieure traverse ledit bouchon tournant (8), ce dispositif comprenant au moins un porte-outil (36) situé à une distance de l'axe de rotation (25) du bouchon (8) égale au rayon de l'un des arcs de cercles définis par les embouts d'aiguilles creuses (17, 18).

5. Banc de prélèvement selon l'une quelconque des revendications 3 ou 4, caractérisé en ce que la cuve (2) comporte à sa périphérie un récipient annulaire (12) rempli d'un liquide dans lequel plonge un manchon (11) solidaire du bouchon tournant (8).

6. Banc de prélèvement selon l'une quelconque des revendications 1 à 5, caractérisé en ce que le cruchon (51) est disposé à l'intérieur d'un curseur (52) permettant le transport pneumatique de l'ensemble cruchon-curseur (40).

7. Banc de prélèvement selon l'une quelconque des revendications 1 à 6, caractérisé en ce que le bouchon élastique (55) du cruchon (51) comprend une cavité entièrement fermée (54) traversée par l'aiguille lors du prélèvement.

8. Banc de prélèvement selon l'une quelconque des revendications 6 ou 7, caractérisé en ce que chaque dispositif mécanique (31) comprend au moins une vis hélicoïdale (32) libre en rotation et immobilisée en translation, permettant le mouvement d'un ensemble cruchon-curseur (40) dans un puits de descente (46), un outil de préhension (39) et un barillet (34) situé à la partie inférieure dudit dispositif mécanique (31) permettant de déplacer l'ensemble cruchon-curseur entre le puits de descente (46) et l'outil de préhension (39).

9. Banc de prélèvement selon l'une quelconque des revendications 1 à 8, caractérisé en ce que chaque embout d'aiguille (17, 18) repose sur un support muni d'une conduite d'arrivée et d'une conduite de départ du liquide à échantillonner ainsi que d'un récipient intermédiaire situé entre les conduites de départ et d'arrivée, la partie inférieure dudit récipient intermédiaire étant reliée à la conduite d'arrivée par une canalisation de vidange fortement inclinée et la partie inférieure de chaque embout (17, 18) ayant la forme d'un manchon plongeant dans le récipient intermédiaire et ouvert à son extrémité inférieure.

**Claims**

1. Apparatus for sampling liquids by means of a receiver or pot (51) closed by an elastic stopper (55) adapted to be pierced by one of a plurality of hollow needles provided with caps (17, 18) connected to a reservoir containing the liquid to be sampled, said reservoir being located beneath a biological protection confinement (4), characterized in that it comprises a closed tank (2) located beneath the protection confinement (4) and unitary therewith, and within which the caps (17, 18) of said hollow needles are located, and at least one mechanical device (31) for manipulation and introduction of said pots (51) in said closed tank (2), the lower part at least of said mechanical device (31) passing through the confinement (4) and abutting within the closed tank (2) at the level of the needle caps (17, 18).

2. Sampling device according to Claim 1, characterized in that the needle caps (17, 18) are disposed in an arc of a circle or of several concentric arcs of circles.

3. Sampling device according to Claim 2, characterized in that the confinement (4) has a rotatable closure (8) whose axis of rotation (25) passes through the centre of said arc or arcs of circles defining the needle caps.

4. Sampling device according to Claim 3, characterized in that it comprises at least one chemical device (31) for manipulation and introduction of the pot (51) whose lower part passes through said rotating closure (8), the device comprising at least one tool carrier (36) located at a distance from the axis of rotation (25) of the closure (8) equal to the radius of one of the arcs of circles defined by the caps of the hollow needles (17,18).

5. Sampling device according to either of Claims 3 or 4, characterized in that the tank (2) has at its circumference an annular receiver (12) filled with a liquid within which dips a sleeve (11) unitary with the rotating closure member (8).

6. Sampling device according to any one of Claims 1 to 5, characterized in that the pot (51) is located within a traveller (52) permitting pneumatic transport of the pot-traveller assembly (40).

7. Sampling device according to any one of Claims 1 to 6, characterized in that the elastic stopper (55) of the pot (51) comprises a completely closed cavity (54) traversed by the needle during sampling.

8. Sampling device according to either of Claims 6 or 7, characterized in that each mechanical device (31) comprises at least one helical screw (32) freely rotatable and immobilized in translation, permitting movement of a pot-traveller assembly (40) in a descent shaft (46), a gripping member (39) and a cylinder (34) located at the lower end of the mechanical device (31) enabling displacement of the pot-traveller assembly between the descent shaft (46) and the gripping member (39).

9. Sampling device according to any one of Claims 1 to 8, characterized in that each needle cap (17, 18) rests on a support having an inlet conduit and an outlet conduit for liquid to be sampled, as well as an intermediate receiver located between the inlet and outlet conduits, the lower part of said intermediate receiver being connected to the inlet conduit by a highly-inclined channel, and the lower part of said cap (17, 18) having the form of a sleeve dipping in the intermediate receiver and open at its lower end.

## Patentansprüche

1. Vorrichtung zur Entnahme von Flüssigkeitsproben mit Hilfe eines Behälters oder Gefäßes (51), welches durch einen elastichen Stopfen (55) verschlossen ist, den man auf eine von mehreren hohlen, mit einem Ansatz (17, 18) versehenen, mit einem die Probenflüssigkeit enthaltenen Behälter verbundenen Nadeln sticht, wobei sich der Behälter unter einer Platte (4) zum biologischen Schutz befindet, dadurch gekennzeichnet, daß die Vorrichtung einen geschlossenen Behälter (2), der sich unter der Schutzplatte (4) befindet und mit dieser fest verbunden ist und in dem sich die Ansätze (17, 18) der hohlen Nadeln befinden, und wenigstens eine mechanische Vorrichtung (31) zur Handhabung und zum Einführen des Gefäßes (51) in den geschlossenen Behälter (2) aufweist, wobei wenigstens ein unterer Abschnitt der mechanischen Vorrichtung (31) die Platte (4) durchquert und in dem geschlossenen Behälter (2) auf der Höhe der Ansätze der Nadeln (17, 18) anstößt.

2. Vorrichtung zur Entnahme nach Anspruch 1, dadurch gekennzeichnet, daß die Ansätze der Nadeln (17, 18) längs eines Kreisbogens oder mehrerer, konzentrischer Kreisbögen angeordnet sind.

3. Vorrichtung zur Entnahme nach Anspruch 2, dadurch gekennzeichnet, daß die Platte (4) einen Drehdeckel (8) aufweist, dessen Drehachse (25) durch die Mitte des Kreisbogens oder der Kreisbögen hindurchgeht, der bzw. die durch die Ansätze der Nadeln festgelegt ist bzw. sind.

4. Vorrichtung zur Entnahme nach Anspruch 3, dadurch gekennzeichnet, daß die Vorrichtung wenigstens eine mechanische Vorrichtung (31) zum Einbringen und Handhaben der Gefäße (51) aufweist, deren unterer Abschnitt den Drehdeckel (8) durchquert, wobei diese Vorrichtung wenigstens eine Werkzeughalterung (36) aufweist, die von der Drehachse (25) des Deckels (8) mit einem Abstand angeordnet ist, der gleich dem Radius eines der Kreisbögen ist, die von den Ansätzen der hohlen Nadeln (17, 18) festgelegt sind.

5. Vorrichtung zur Entnahme nach einem der Ansprüche 3 oder 4, dadurch gekennzeichnet, daß der Behälter (2) an seinem Umfang einen mit einer Flüssigkeit gefüllten Ringbehälter (12) umfaßt, in den eine mit dem Drehdeckel (8) fest verbundene Hülse (11) taucht.

6. Vorrichtung zur Entnahme nach einem der Ansprüche 1 bis 5, dadurch gekennzeichnet, daß das Gefäß (51) im Inneren eines Läufers (52) angeordnet ist, wodurch ein pneumatischer Transport der Gesamtheit Gefäß-Läufer (40) durchführbar ist.

7. Vorrichtung zur Entnahme nach einem der Ansprüche 1 bis 6, dadurch gekennzeichnet, daß der elastische Stopfen (55) des Gefäßes (51) einen vollständig geschlossenen, von der Nadel bei der Entnahme durchquerten Hohlraum (54) aufweist.

8. Vorrichtung zur Entnahme nach Anspruch 6 oder 7, dadurch gekennzeichnet, daß jede mechanische Vorrichtung (31) wenigstens eine frei drehbare und unverschiebbare spiralförmige Schraube (32), mit der eine Gesamtheit Gefäß-Läufer (40) in einem Absenkschacht (46) bewegbar ist, ein Greifwerkzeug (39) und ein tonnenförmiges Gehäuse (34) aufweist, welches an dem unteren Abschnitt der mechanischen Vorrichtung angeordnet ist und mit dem die Gesamtheit Gefäß-Läufer zwischen dem Absenkschacht (46) und dem Greifwerkzeug (39) bewegbar ist.

9. Vorrichtung zur Entnahme nach einem der Ansprüche 1 bis 8, dadurch gekennzeichnet, daß jeder Ansatz der Nadel (17, 18) auf einer Stütze ruht, die mit einer Zuführleitung und einer Abführleitung für die Probenflüssigkeit sowie mit einem Zwischenbehälter versehen ist, der zwischen der Zuführ- und der Abführleitung angeordnet ist, daß der untere Abschnitt des Zwischenbehälters mit der Zuführleitung über eine stark geneigte Entleerungsleitung verbunden ist und daß der untere Abschnitt von jedem Ansatz (17, 18) die Form einer Hülse aufweist, die in den Zwischenbehälter taucht und an ihrem unteren Ende offen ist.

FIG.1

FIG.2

FIG.3

FIG.4

FIG.5

4

FIG. 6

FIG. 7